# FASCICULE DE BREVET EUROPEEN

(11) **EP 2 694 021 B1**
(45) Date de publication et mention de la délivrance du brevet: **07.10.2015**
(21) Numéro de dépôt: 12720242.2
(22) Date de dépôt: 05.04.2012
(51) Int. Cl.: A61K 8/39, A61K 8/86, A61K 8/34, A61K 8/81, A61Q 19/00, A61K 9/00, A61K 45/06, A61K 9/06, A61K 8/04, A61K 31/78

(54) **Composition cicatrisante pour application topique**
Heilende Zusammensetzung zur topischen Anwendung
Healing composition for topical application

(30) Priorité: 06.04.2011 FR 1152997
(43) Date de publication de la demande: 12.02.2014
(73) Titulaire: Biopass S.A., 2227 Luxembourg (LU)
(72) Inventeur: GREFF, Daniel, F-78490 Mere (FR)
(74) Mandataire: Chantraine, Sylvie Hélène
(86) Numéro de dépôt international: PCT/FR2012/050735
(87) Numéro de publication internationale: WO 2012/136934

(56) Documents cités:
- EP-A1- 1 238 651
- EP-A1- 1 543 825
- WO-A1-97/47310
- FR-A1- 2 682 296
- FR-A1- 2 737 406
- FR-A1- 2 787 709
- FR-A1- 2 854 897
- DATABASE WPI Week 201038 Thomson Scientific, London, GB; AN 2010-G06054 XP002662852, & JP 2010 120859 A (MANDOM KK) 3 juin 2010 (2010-06-03)

## Description

L'invention concerne une composition pour application topique, en particulier une composition dermatologique ou pharmaceutique sous forme d'un hydrogel présentant des propriétés cicatrisantes. La composition de l'invention est particulièrement efficace dans la prise en charge des lésions cutanées aigües - comme les plaies - et des lésions cutanées chroniques, comme les ulcères et les cicatrices.

La peau est constituée du derme et de l'épiderme. L'épiderme assure, de par sa structure, la fonction de barrière d'étanchéité de l'organisme vis-à-vis du monde extérieur. L'épiderme est organisé en différentes couches, la couche basale, la couche épineuse (stratum spinosum), la couche granuleuse (stratum granulosum), le stratum lucidum et la couche cornée (stratum corneum). Par lésion cutanée aiguë au sens de l'invention, on entend la destruction de tout ou partie du revêtement cutané survenant à la suite d'un traumatisme, qu'il s'agisse de plaies accidentelles ou chirurgicales, de coupures, ou de lésions de frottement (avulsions). La lésion peut être plus ou moins étendue et plus ou moins profonde, selon qu'elle atteint une grande surface de la peau ou non.

### ETAT DE L'ART

Les demandes de brevets FR 2 737 406 et WO 97/30692, et WO 97/05856 décrivent des gels aqueux associant des diols, des polymères polyacrylate et de la glycérine. Ces gels présentent une activité antimicrobienne particulièrement élevée et sont proposés comme substituants d'agents conservateurs dans des produits pharmaceutiques, cosmétiques ou dermatologiques. Ces documents ne décrivent aucune propriété biologique que ces gels pourraient avoir sur la peau. Ils sont simplement utilisés comme agent conservateur dans des produits finis.

La demande WO 97/474310 décrit quant à elle les propriétés anti-inflammatoires et hydratantes de certains de ces gels. Par exemple, un gel constitué de 1% en poids de polyacrylate, de 34% en poids d'eau et de 65% en poids de glycérine est efficace dans la prévention d'un érythème et d'une sécheresse cutanée qui peuvent apparaître suite à des séances de radiothérapie. Leur utilisation dans la prévention des brûlures légères, c'est-à-dire non ouvertes, que l'on qualifie également de brûlures du premier degré ou du deuxième degré léger est suggérée sur la base de leur pouvoir antiseptique et hydratant.

La plupart des exemples de formulation donnés dans ce document contiennent de l'éthoxydiglycol. Or, il a été découvert dans le cadre de la présente invention que ce composé retarde de façon significative la cicatrisation des plaies. Sa présence dans un gel empêche donc le traitement de réussir sur une peau dont une partie de l'épiderme ou du derme a été détruit.

Par ailleurs, certains produits qui ont été développés en suivant les enseignements du document WO 97/474310 se sont révélés, sur la base d'études cliniques, inadaptés pour le soin des brûlures graves (telles que les brûlures du deuxième degré profond ou les brûlures du troisième degré), des brûlures surinfectées ou des peaux écorchées. Aussi, contrairement aux suggestions de ce document, aucun des gels qu'il ne décrit n'est adapté au traitement des lésions cutanées aiguës ou chroniques.

Il a également été décrit que des gels à base de polyacrylate, d'eau et de glycérine sont dotés de propriétés hydratantes des couches superficielles de la peau et peuvent être utilisés dans des lignes de soin pour peaux sensibles ou pour peaux sèches. Par exemple, la plupart des exemples de formulation donnés dans le document WO 97/474310 contiennent de fortes proportions de glycérine. Un mode de réalisation présenté comme préféré est composé de 30-60% de glycérine, 30-50% d'eau et 0,1-5% de polymère. Les propriétés hydratantes de ces gels découlent de la forte proportion de glycérine qu'ils contiennent. En effet, la glycérine est une petite molécule capable de pénétrer dans les cellules superficielles de l'épiderme, de capter au moins en partie l'eau présente dans la composition cosmétique ou pharmaceutique, et de la retenir dans les cellules grâce à ses propriétés hygroscopiques. Les gels de polyacrylate et de glycérine sont donc connus pour leurs propriétés d'hydratation de surface d'une peau saine.

### BUTS DE L'INVENTION

L'invention a pour but principal de résoudre le problème technique consistant en la fourniture d'une composition pharmaceutique cicatrisante pour le soin topique des lésions cutanées aiguës ou chroniques, et des lésions mucosales.

L'invention a pour but de fournir des compositions pharmaceutiques garantissant une cicatrisation particulièrement efficace des plaies de la peau ou des muqueuses. La présente invention a pour but en particulier de fournir une composition dont l'activité cicatrisante est au moins comparable, voire améliorée, par rapport aux compositions cicatrisantes de l'art antérieur.

L'invention a encore pour but de fournir de telles compositions de manière reproductible à l'échelle industrielle, peu coûteuses et fiables du point de vue de leur acceptation topique au niveau de la peau et des muqueuses.

### DESCRIPTION DE L'INVENTION

Il a été découvert que les compositions de la présente invention présentent des propriétés cicatrisantes particulièrement intéressantes allant au-delà des propriétés espérées. La composition de l'invention permet une cicatrisation au moins égale, voire supérieure à celle de certaines compositions de référence de l'art antérieur.

La cicatrisation au sens de l'invention englobe les processus par lesquels la peau ou la muqueuse referme et répare toute discontinuité dans sa structure après une agression qui conduit à une lésion profonde (par opposition à superficielle). Ces agressions incluent par exemple une coupure, une ouverture volontaire faite pour une intervention chirurgicale, ou des ulcères dermiques. Les lésions profondes se distinguent des lésions superficielles qui ne touchent que l'épiderme. L'évolution et les perturbations des lésions profondes posent de délicats problèmes thérapeutiques qui ne se posent pas avec les lésions superficielles.

La cicatrisation comprend quatre étapes dont la coagulation du sang, un gonflement inflammatoire, la détersion, et enfin la prolifération de nouveaux tissus qui permet selon les cas l'accolement des berges de la plaie ou un bourgeonnement qui reconstruit l'épithélium. Ce mécanisme aboutit à la formation d'une cicatrice de plus ou moins grande qualité esthétique, selon la profondeur et la largeur de la lésion initiale.

Dans les cas de lésions profondes, la cicatrisation répond à un mécanisme complexe: après la réaction inflammatoire immédiate, le comblement de la perte de substance se fait par un tissu transitoire jeune et lâche, le bourgeon charnu ou tissu de granulation. Ce tissu contient de nombreux macrophages et fibroblastes qui sécrètent différents composants de la matrice extracellulaire (glycosaminoglycanes, fibronectine et collagène). Un important réseau vasculaire capillaire se développe au sein de ce tissu de granulation. Certains fibroblastes, les myofibroblastes, présentent des caractéristiques comparables à celles des cellules musculaires lisses et sont capables de contraction. Ils sont à l'origine d'une rétraction des berges qui aide à la fermeture de la plaie. Lorsque le bourgeon a comblé la perte de substance la réparation épidermique se fait soit à partir des berges de la lésion, soit à partir des reliquats épidermiques sains au niveau de la plaie. Après la migration transversale aboutissant à la fermeture de la plaie, on observe une prolifération verticale avec différenciation et maturation des kératinocytes en un épithélium pluristratifié puis la reconstruction des différents types cellulaires de l'épidémie.

Une fois la plaie fermée, l'évolution secondaire est longue et variable avant d'accéder à la maturation cicatricielle à partir de laquelle la cicatrice n'est plus inflammatoire et n'évolue plus. On comprend bien en quoi le mécanisme de cicatrisation des plaies superficielles diffèrent du mécanisme de cicatrisation des plaies profondes. Les moyens de les soigner ne peuvent donc être identiques.

Pendant toute la phase précédant la maturation cicatricielle, on observe une persistance de l'inflammation du derme avec hypervascularisation et oedème, dépôt anarchique et non orienté de collagène III, présence de myofibroblastes et dépôts de tissus fibreux. Les dépôts de tissus fibreux et de collagène peuvent épaissir considérablement le derme et laisser des cicatrices hypertrophiques ; parfois ces dépôts aboutissent à de véritables tumeurs cutanées, les chéloïdes.

L'amélioration de la cicatrisation des plaies apportée par les compositions de la présente invention peut se traduire par une accélération de la cicatrisation : le délai de cicatrisation complète est diminué. Elle peut aussi se traduire par une diminution de la douleur que les plaies peuvent provoquer lors de la cicatrisation. Ce résultat est d'autant plus surprenant que l'art antérieur n'a mentionné que les propriétés d'hydratation des gels de glycérine et de polyacrylate sur les couches superficielles de la peau.

Les compositions de l'invention permettent également avantageusement d'éviter ou de limiter la formation de cicatrices et de taches achromiques inesthétiques qui peuvent subsister après cicatrisation complète de la plaie.

Les compositions de l'invention permettent notamment une meilleure épithélialisation de la lésion et une diminution significative de la taille de la lésion. Elles augmentent le degré d'épithélialisation de lésions profondes. Elles permettent également la ré-épithélialisation d'ulcères dermiques chroniques qui ne pouvait plus naturellement s'opérer.

Il a été découvert de façon tout à fait inattendue que des gels de polyacrylate et de glycérine particuliers permettent de promouvoir la cicatrisation des plaies ou des lésions cutanées - chroniques ou aiguës - qui sont plus ou moins profondes et pour lesquelles l'épiderme est quasiment détruit.

La composition de l'invention permet avantageusement de cicatriser une plaie chronique ou aiguë plus rapidement, en minimisant les souffrances du patient et en évitant des complications cicatricielles. Cet objectif est atteint pour la première fois avec une thérapeutique topique.

### COMPOSITION

Ainsi, la présente invention concerne une composition pharmaceutique pour application topique sur la peau ou les muqueuses contenant moins de 0.1% en poids d'éthoxydiglycol et comprenant de l'eau, de 5 à 25 % en poids de glycérol, de 0.01 à 2% en poids d'au moins un polymère poly(méth)acrylate, de 0.5 à 5% en poids d'au moins un polyéthylène glycol de masse moléculaire inférieure à 1000 g/mol , de 0.1 à 1.5% en poids d'octanediol, les pourcentages en poids étant exprimés par rapport au poids total de la composition.

La composition comprend avantageusement de 5 à 25 % en poids de glycérol (encore appelé glycérine), de préférence de 5 à 20% en poids, et plus préférentiellement de 5 à 10% en poids de glycérol par rapport au poids total de la composition.

La composition comprend avantageusement de 0.5 à 5% en poids de polyéthylène glycol, de préférence de 1 à 5% en poids, et plus préférentiellement de 2 à 4% en poids de polyéthylène glycol par rapport au poids total de la composition. La masse moléculaire en poids du polyéthylène glycol est avantageusement inférieure à 1000 g/mol, de préférence comprise entre 200 et 600 g/mol, par exemple de l'ordre de 400 g/mol.

Un polyéthylène glycol est de préférence de faible poids moléculaire, de préférence choisi parmi les polyéthylènes de masse moléculaire inférieure à 500 g/mol, comme le PEG-8 (encore appelé octaéthylène glycol). On peut également utiliser les polyéthylènes glycol de dénomination courante PEG 200, PEG 400, PEG 600, ou PEG 1000 (dont le chiffre correspond à une masse moléculaire moyenne en poids).

Le polymère poly(méth)acrylate est par exemple présent dans la composition en une quantité de 0.1 à 2% en poids par rapport au poids total de la composition.

Comme polymère poly(méth)acrylate, on utilise de préférence un polymère sous forme de sel, et de préférence de sel de métal alcalin comme les sels sodiques ou potassiques. On utilise par exemple comme poly(méth)acrylate un produit de la gamme Carbopol^{®} de la Société Goodrich. On peut utiliser de façon avantageuse un mélange de plusieurs poly(méth)acrylates, comme le mélange d'un polyméthacrylate de méthyle, de polymère réticulé acrylate/acrylate de C10-30 alkyle (nom INCI Carbomer) et de polyacrylate de sodium.

La composition comprend de préférence de 0.1 à 1.5% en poids d'octanediol, de préférence de 0.5 à 1.5% en poids par rapport au poids total de la composition. Le ratio massique entre la glycérine et l'octanediol est de préférence compris entre 5 :1 et 15 :1, de préférence encore entre 8 :1 et 10 :1.

Un octanediol préféré est le 1,2 octanediol, encore appelé caprylyl glycol.

Selon un mode de réalisation particulier, la composition de la présente invention est constituée de l'association du glycérol, d'au moins un polymère poly(méth)acrylate, d'au moins un polyéthylène glycol de poids moléculaire inférieur à 1000 g/mol, d'octanediol, d'eau, et éventuellement d'un ingrédient actif sur la cicatrisation de la peau connu de l'homme du métier. Les caractéristiques qui ont été décrites plus haut, notamment les teneurs des différents composants de la composition, s'appliquent toute à ce mode de mise en oeuvre particulier.

La composition de la présente invention contient moins de 0,1% en poids pas d'éthoxydiglycol, et de préférence n'en contient pas, car il a été découvert dans le cadre de la présente invention que ce composé interfère avec les processus biologiques de la peau. Il retarde en particulier de façon significative la cicatrisation de plaies. Sa présence dans la composition empêche donc le traitement de réussir.

Les concentrations des produits de la composition de la présente invention sont indiquées en fonction de la formulation finale qui dépend de l'application souhaitée.

La composition de la présente invention est de préférence sous forme de gel ou d'hydrogel.

Une composition sous forme d'hydrogel selon l'invention ayant une composition particulièrement avantageuse comprend de l'eau, de 5 à 25 % en poids de glycérol; de 0.01 à 2% en poids de poly(méth)acrylate; de 0.5 à 5% en poids d'un polyéthylène glycol et de 0.1 à 1.5% en poids de 1,2 octanediol, par rapport au poids total de la composition.

Selon une variante, la composition de l'invention est uniquement constituée du glycérol; d'au moins un polymère poly(méth)acrylate; d'au moins un polyéthylène glycol de poids moléculaire inférieur à 1000 g/mol; d'au moins un octanediol; et d'eau.

Dans un mode de mise en oeuvre, la composition de l'invention est constituée d'eau, de 5 à 25 % en poids, de préférence de 5 à 10% en poids, de glycérol; de 0.01 à 2% en poids de poly(méth)acrylate; de 0.5 à 5% en poids, de préférence de 1 à 5% en poids, d'un polyéthylène glycol, et de 0.1 à 1.5% en poids, de préférence de 0.5 à 1.5% en poids, de 1,2 octanediol, les pourcentages étant exprimés par rapport au poids total de la composition.

### TRAITEMENTS

Il a été découvert - de façon tout à fait inattendue et contrairement à l'enseignement de l'art antérieur - que des gels de polyacrylate et de glycérine particuliers permettent de promouvoir la cicatrisation des plaies ou des lésions cutanées chroniques et aiguës. Parmi les plaies chroniques, on pourra citer les escarres, les ulcères, en particulier les ulcères du pied diabétique. Parmi les plaies aiguës, on pourra citer les brûlures du deuxième degré profond, les brûlures du troisième degré, la dermabrasion, les plaies traumatiques, et les plaies post-opératoires.

Les plaies cutanées chroniques ou aiguës au sens de l'invention sont des lésions profondes touchant l'intégrité de la structure de la peau ou de la muqueuse, au-delà de la couche granuleuse, de préférence jusqu'au niveau de la couche basale. Les plaies profondes atteignent généralement le derme sur une partie ou la totalité de son épaisseur.

La composition de l'invention est particulièrement utile pour le traitement des lésions pour lesquelles la quasi-totalité de l'épiderme a été détruite. Elle est même efficace sur des plaies pour lesquelles la peau est entièrement détruite et qui ne peuvent cicatriser qu'à partir des berges de la lésion ou par réalisation d'une greffe cutanée.

Dans les plaies superficielles, comme les brûlures du deuxième degré superficiel, la perte du stratum corneum et de son film lipidique de surface est responsable de la mort secondaire de nombreuses cellules épidermiques. Le maintien d'une hydratation de surface est suffisante pour guérir ces plaies. Ce n'est pas le cas des brûlures plus graves.

Les compositions de l'invention sont avantageusement utilisées pour le traitement des plaies cutanées des personnes diabétiques et des personnes âgées (ayant plus de 60 ans), chez qui les troubles de la cicatrisation sont plus fréquents.

Un ulcère au sens de l'invention est une lésion de la peau ou des muqueuses caractérisée par une perte de substance dermique. Les ulcères peuvent entraîner une perte complète de portions plus ou moins importantes d'épiderme et de derme et même souvent de graisse sous-cutanée.

Par exemple la composition de l'invention est particulièrement efficace pour améliorer la cicatrisation des bulles dermiques ulcérées qui peuvent être provoquées par des pathologies comme la varicelle ou l'herpès. Les bulles sont des lésions cutanées de grande taille (supérieur à 5 mm environ) provoquées par un décollement entre les différentes couches de la peau (épiderme, derme, hypoderme). Les bulles peuvent s'ulcérer ou laisser des cicatrices, c'est pourquoi, il est essentiel de les soigner. La composition de l'invention trouve tout son intérêt dans le traitement cicatrisant préventif ou curatif de la cicatrisation des boutons de varicelle et d'herpès, notamment de l'herpès labial.

Les ulcères de la peau ont la forme de cratères ouverts, souvent ronds ou ovales. Ils laissent apparaitre sur leurs bords des couches de peau qui sont érodés. La peau entourant l'ulcère peut être rougie, enflée et sensible voire douloureuse. Un liquide (mélange de lymphe et moindrement de sang ou de pus parfois) peut suinter de l'ulcère. Les symptômes de l'ulcère chronique incluent généralement une douleur croissante, une plaie granuleuse, friable, avec rupture de la plaie au lieu d'une guérison. Les ulcères qui cicatrisent en 12 semaines ou moins sont généralement classés comme « actifs », et ceux qui cicatrisent plus lentement sont dits « chroniques ».

Ils sont plus fréquents sur la peau des membres inférieurs mais peuvent aussi apparaître sur les joues, le nez, le palais mou, la langue, et à l'intérieur de la lèvre inférieure. Ces petits ulcères de la bouche durent habituellement de 7 à 14 jours et peuvent être douloureux.

La composition de l'invention permet de traiter efficacement les ulcères, en particulier les ulcères chroniques, en évitant l'infection de l'ulcère, en limitant la perte de tissus par nécrose, en limitant les suintements, en contrôlant l'oedème, et en soulageant la douleur induite par les lésions des tissus et des nerfs.

Parmi les ulcères, on pourra citer les ulcères de pression. Un manque de mobilité (alitement, fauteuil roulant) induit une pression prolongée sur certains tissus, qui limite la circulation sanguine et lymphatique, ce qui favorise des ulcères de la peau, communément appelés escarres.

L'ulcération des plaies est plus fréquente sur les membres inférieurs et les extrémités du corps particulièrement vulnérables aux désordres circulatoires aboutissant à une ou plusieurs lésions de peau et entravant la cicatrisation normale de ces dernières.

Les compositions de la présente invention sont particulièrement efficaces pour lutter contre les ulcères dermiques, notamment les ulcères dermiques chroniques situés sur les membres inférieurs.

Il faut noter que l'ulcère dermique, par exemple au niveau de la jambe, est une perte de substance cutanée épidermique et dermique d'évolution chronique en l'absence de traitement. Plus de 90% des ulcères sont d'origine vasculaire. L'étiologie veineuse est la plus fréquente. Le problème majeur est celui d'une absence ou d'une cicatrisation particulièrement délicate.

L'ulcère veineux survient lors d'une insuffisance veineuse des membres inférieurs parfois consécutif à une phlébite ou à des varices. L'ulcère artériel de jambe survient en cas de trouble circulatoire artériel, par exemple suite à une consécutive à l'athérosclérose. Les ulcères veineux représentent 90% des ulcères de la jambe, et sont généralement des ulcères variqueux (post thrombotiques) ou dus à une insuffisance veineuse chronique (chez les personnes âgées ou diabétiques particulièrement).

Un ulcère peut également survenir suite aux piqûres ou aux morsures d'animaux capables d'injecter un venin contenant des toxines et des enzymes nécrosantes. La composition de l'invention est ainsi particulièrement utile pour le soin des piqûres ou des morsures d'animaux venimeux comme les guêpes, les abeilles, les taons ou les méduses.

La composition de l'invention permet également de cicatriser les plaies des muqueuses. Les muqueuses recouvrent les parois du tube digestif de la bouche à l'anus, les parois de l'appareil respiratoire comme les narines ou le canal auditif, les parois du vagin et le téton des seins. Les plaies des muqueuses buccale, labiale, nasale sont efficacement cicatrisées grâce à la composition de l'invention.

L'invention a encore pour objet la composition décrite précédemment pour le traitement cicatrisant des fissures anales, le traitement cicatrisant des blessures de la muqueuse buccale (comme les aphtes ou les écorchures provoquées par un appareil dentaire), le traitement cicatrisant des lèvres (notamment le traitement cicatrisant de l'herpès labial communément appelé bouton de fièvre).

Au niveau de la muqueuse buccale, des ulcères - appelés communément aphtes - se forment sur l'intérieur des lèvres et des joues, sur la langue, le palais, les gencives ou la gorge. Les blessures ou les irritations à l'intérieur de la bouche sont généralement occasionnées par un mauvais ajustement de prothèses dentaires, par une chirurgie buccale, par un usage trop énergique de la brosse à dents ou par un mordillement de la joue, qui peuvent provoquer l'apparition d'aphtes ou leur aggravation.

La composition peut également être utilisée pour la cicatrisation des lésions qui apparaissent à l'intérieur des narines, en cas de rupture capillaire. A titre préventif, elle peut avantageusement servir de décongestionnant nasal, ou pour l'hygiène de l'oreille.

La composition de l'invention permet également de prendre en charge le traitement des crevasses ou des fissures, qui apparaissent fréquemment sur les extrémités du corps (talon des pieds, entre les orteils, doigts) ou sur les tétons des seins en cas d'allaitement.

Les cicatrices définitives se distinguent des cicatrices transitoires qui ne restent inflammatoires et douloureuses que quelques jours après la fin du processus de cicatrisation. La composition de l'invention permet avantageusement de limiter ou d'empêcher la maturation des cicatrices en cicatrice hypertophiques ou en chéloïdes.

Avantageusement, la composition de la présente invention permet de mettre en oeuvre d'autres traitements choisis parmi le groupe consistant en :
- le traitement cicatrisant des dermites, notamment des dermites de contact des mains, des dermites séborrhéiques du visage, des dermites et des démangeaisons du cuir chevelu,
- le traitement cicatrisant et apaisant des eczémas et des états psoriasiques, et
- le traitement cicatrisant des érythèmes fessiers.

Selon un mode de réalisation particulier, la composition de l'invention est sous la forme d'un gel conditionné dans un tube.

La composition peut être appliquée sur la peau puis recouverte d'un pansement occlusif. Elle peut également être appliquée sur une gaze ou un non tissé qui aura été placé préalablement sur la plaie.

Selon un mode de réalisation particulier, l'invention concerne un pansement occlusif comprenant la composition de l'invention.

L'invention a également pour objet un pansement prêt à l'emploi, qui ne nécesssite pas la pose d'une gaze et d'une bande, comprenant dans sa structure la composition qui vient d'être décrite. Un pansement au sens de l'invention comprend au moins deux couches, une couche interne destinée à entrer en contact avec la peau et une couche externe protectrice. La composition de l'invention entre par exemple dans la consitution de la couche interne. La couche externe du pansement est généralement un film ou une mousse en matière polymère, par exemple en polyuréthane ou en polyester/polyamide. La structure du pansement peut comprendre une couche comprenant un matériau textile non tissé, tricoté ou tissé, par exemple une compresse.

Selon un mode de réalisation, la composition ou le pansement la comprenant est appliquée sur une plaie causée par une intervention chirurgicale ou dermatologique, notamment afin d'aider la cicatrisation de la peau ou de la muqueuse.

L'invention a également pour objet un produit d'hygiène contenant moins de 0.1% en poids d'éthoxydiglycol et comprenant de l'eau, de 5 à 25 % en poids de glycérol, de 0.01 à 2% en poids d'au moins un polymère poly(méth)acrylate, de 0.5 à 5% en poids d'au moins un polyéthylène glycol de masse moléculaire inférieure à 1000 g/mol , de 0.1 à 1.5% en poids d'octanediol, les pourcentages en poids étant exprimés par rapport au poids total de la composition. Ce produit d'hygiène peut être utilisé comme gel de rasage ou comme baume après rasage pour peaux sensibles ou ultrasensibles, comme décongestionnant nasal, ou pour l'hygiène de l'oreille.

Le produit d'hygiène de l'invention permet une bonne qualité de rasage, une cicatrisation quasi immédiate des irritations dues au rasage et un pouvoir antidouleur du au "feu" du rasoir.

La présente invention concerne selon un autre aspect une méthode de traitement thérapeutique qui consiste à appliquer à un sujet en ayant besoin, notamment un être humain, une composition telle que décrite précédemment sur une lésion choisie dans le groupe constitué des plaies cutanées aiguës, des plaies cutanées chroniques, et des lésions situées sur les muqueuses.

Ce type de plaies cutanées se distingue de plaies plus superficielles dans lesquelles le derme n'est pas atteint et une partie seulement de l'épiderme a été endommagée.

Selon cette méthode, la composition est appliquée sur une peau cicatricielle, une plaie de la peau, notamment la peau du visage, la peau des jambes ou le cuir chevelu.

L'invention a encore pour objet une méthode de traitement qui consiste appliquer la composition décrite précédemment sur une zone de la peau ulcérée, dans laquelle l'épiderme a été détruit au moins partiellement. Selon un mode de réalisation, la composition peut être utilisée pour la cicatrisation d'une plaie dans laquelle au moins une partie du derme est endommagée.

L'invention a encore pour objet une méthode de traitement qui consiste à appliquer la composition décrite précédemment sur une plaie ouverte ou sur un plaie occasionnée par une intervention chirurgicale.

Toutes les caractéristiques qui ont été décrites précédemment en relation avec la composition de l'invention s'appliquent à la méthode de traitement selon l'invention également.

D'autres buts, caractéristiques et avantages de l'invention apparaîtront clairement à l'homme de l'art suite à la lecture des exemples suivants.

### EXEMPLES

### Exemple 1: Traitement cicatrisant de plaies aiguës ou chroniques

Un essai clinique non comparatif prospectif à centre unique a été effectué sur des patients volontaires atteints de différents types de lésions cutanées. L'âge moyen était de 60,3 ans. Les causes des problèmes de cicatrisation étaient dues principalement à des ulcères de pression (6 cas), des cicatrices du pied (7 cas), des ulcères variqueux (10 cas), des brûlures du deuxième degré profond (2 cas), des dermatoses bulleuses des jambes (3 cas), une fissure entre les orteils (2 cas) et un ulcère traumatique (2 cas). Les membres inférieurs étaient la principale localisation (80 %).

La composition qui a été appliquée était un hydrogel composé d'eau, de 0,5-1,5 % en poids de 1,2-octane-diol, de 5-10% en poids de glycérol, de 1-5% en poids de polyéthylène glycol de masse moléculaire de l'ordre de 400 g/mol, de polyméthacrylate de méthyle, de polymère réticulé acrylate/acrylate de C10-30 alkyle et de polyacrylate de sodium.

Le produit a été appliqué quotidiennement de manière épaisse après avoir bien nettoyé la plaie avec du sérum physiologique et l'avoir recouverte d'une compresse stérile. L'application de tout autre produit sur la zone étudiée a été évitée pendant toute la durée de l'essai.

Protocole d'évaluation : L'évolution de la cicatrice a été évaluée par iconographie prise chaque semaine dans les 2 premières semaines, puis tous les mois. Elle a été évaluée par le patient et le médecin en même temps. Le principal point final était le niveau de ré-épithélialisation, l'appréciation de l'efficacité et de l'innocuité du produit (par le médecin) et le temps de disparition de la douleur, du suintement et de l'érythème (par le médecin et le patient).

Résultats : Les résultats observés sur une partie des patients n'ont pas été pris en compte en raison de la non observance de l'application.

Le délai de disparition de l'érythème, de la douleur et du suintement était 1 semaine dans 80 % des cas. Le début de la guérison a été noté dans la deuxième semaine chez 50 % des sujets. Le traitement a été appliqué pendant 3 mois dans 50 % des cas. L'application a été prolongée pendant plus de 4 mois dans 4 cas. Il n'y avait aucun effet secondaire noté et la tolérance a été jugée bonne à très bonne dans 100 % des cas.

### Exemple 2 : Evaluation de l'efficacité cicatrisante sur des lésions post intervention dermatologique

Une étude randomisée comparative à la pommade HYALUZINC^{®} a été réalisée. Cette étude a été conduite auprès de 2 groupes de 22 volontaires présentant une lésion après intervention avec de l'azote liquide sur des kératoses ou des taches pigmentaires. Les signes cliniques sont évalués à J0, J12-J15 et J28 (notamment la réépithélialisation de la lésion, la taille de la lésion, le délai de cicatrisation).

La composition de l'invention se présentait comme un hydrogel composé d'eau, de 0,5-1,5 % en poids de 1,2-octane-diol, de 5-10% en poids de glycérol, de 1-5% en poids de polyéthylène glycol de masse moléculaire de l'ordre de 400 g/mol, de polyméthacrylate de méthyle, de polymère réticulé acrylate/acrylate de C10-30 alkyle, et de polyacrylate de sodium.
- à J12-J15 le degré de réépithélialisation de la lésion était de 82% contre 70% pour le HYALUZINC^{®} (témoin positif)
- La souplesse de la peau était importante ou moyenne chez 90 % des sujets traités par la composition de l'invention.
- Absence de cicatrice résiduelle et de tache achromique résiduelle chez 100% des sujets traités par la composition de l'invention.
- Le délai de cicatrisation complète de la lésion était de 11 jours pour la composition de l'invention et de 13.2 jours pour le témoin positif.

Ces résultats démontrent une activité cicatrisante au moins comparable, voire supérieure à celle d'une composition cicatrisante de référence HYALUZINC^{®}.

### Exemple 3 : Traitement cicatrisant des fissures anales

Pour cette étude, il a été effectué un traitement de fissures anales de première ou de deuxième intention réalisée sur 30 sujets avec la composition de l'exemple 1 comparativement à un témoin positif (Pommade HEC).

Les résultats obtenus après 3 semaines de traitement démontrent de façon nette pour le produit de l'invention, une meilleure cicatrisation, y compris dans les fissures récidivantes de deuxième intention. La cicatrisation est complète dans 80% des cas.

Dans tous les cas il y a une diminution nette des douleurs associées (supérieure à 80%).

### Exemple 4 : Traitement cicatrisant des blessures en bouche

Une étude a été réalisée sur 7 cas avec la composition de l'exemple 1 sur des personnes ayant des aphtes

Le pouvoir cicatrisant de la composition de l'invention permet une bonne résorption des blessures de 80% et une diminution rapide des douleurs associées de 90%.

### Exemple 5 : Cicatrisation des boutons d'herpès labial

Cet exemple concerne un test réalisé sur 10 volontaires présentant un herpès labial (bouton de fièvre) par application locale de la composition de l'exemple 1.

Les résultats observés sont un dégonflage de la bulle, sous quelques heures après la première application, et suite à plusieurs applications un arrêt progressif de l'inflammation et une cicatrisation consécutive rapide (2 à 5 jours selon l'importance de la lésion).

### Exemple 6 : Décongestionnant nasal

Etude réalisée sur 25 personnes présentant des symptômes de « nez bouché » de manière équivalente des deux côtés.

La composition de l'invention est appliquée localement et répartie sur la cavité nasale gauche, la cavité nasale droite servant de témoin.

Le résultat se traduit par un effet décongestionnant immédiat de la narine traitée par rapport à la non traitée (supérieur à 90%). Cet état persiste selon l'affection au moins 20 à 30 minutes. L'opération est alors renouvelée avec le même résultat. De plus, en parallèle on observe une diminution des rougeurs et de la douleur (supérieur à 80%) du côté traité.

### Exemple 7 : Cicatrisation des piqûres de guêpes, taons et méduses

Une étude a été réalisée sur 5 cas de piqûres de méduses, 5 cas de piqûres de taons, et 10 cas de piqûres de guêpes.

Dans le cas des taons et des guêpes, dans 100% des cas, la douleur a disparu entre 1 à 3 minutes maximum. Il n'y avait ni inflammation, ni rougeur et la trace de piqûre a disparu en quelques heures.

Concernant les piqûres de méduses de type *Pelagianoctiluca,* espèce méditerranéenne très urticante, si la composition était appliquée moins de 2 minutes après la piqûre, la douleur disparaissait entre 5 et 10 minutes.

L'inflammation est minimale et les traces de la piqûre ne perdurent pas au-delà de 48 heures dans tous les cas de piqûres.

### Exemple 8 : Traitement cicatrisant des dermites séborrhéiques du visage

Cette étude a été réalisée avec 15 volontaires présentant des dermites séborrhéiques du visage sur 4 semaines.

La composition de l'exemple 1 a été appliquée sous forme d'hydrogel. Ce gel a diminué de façon statistiquement significative les lésions inflammatoires ( -26%), il a permis de refroidir et de réhydrater l'épiderme, permis un resserrement des pores distendus (-15%) et une diminution des comédons (-18%). On a assisté à une diminution des rougeurs associée à une bonne cicatrisation des lésions avec disparition des squames et croutes jaunâtres.

### Exemple 9 : Traitement cicatrisant et apaisant des eczémas et des états psoriasiques

Une étude a été réalisée sur 10 cas présentant des eczémas et sur 5 cas en états psoriasiques en appliquant la composition de l'exemple 1 pendant une durée de quatre semaines.

Les douleurs et les suintements ont diminué de 90% après 4 à 6 applications. Dans les deux types d'affection, on peut noter la diminution des rougeurs de 80% après dix jours de traitement.

Ici encore, l'effet cicatrisant et apaisant après une application locale d'une composition selon l'invention pour lutter contre un eczéma ou un état psoriasique s'est révélé particulièrement efficace.

### Exemple 10 : Traitement cicatrisant des érythèmes fessiers de l'enfant et de l'adulte

Une étude portant sur 23 cas a démontré de façon évidente les résultats favorables de la composition de l'invention. En effet, après 12 à 48 heures selon l'importance de l'érythème, celui-ci a disparu à près de 95%, il était toujours accompagné par une bonne cicatrisation et une disparition de la douleur.

## Revendications

1. Composition pharmaceutique pour application topique sur la peau ou les muqueuses contenant moins de 0.1% en poids d'éthoxydiglycol et comprenant :
de l'eau, de 5 à 25 % en poids de glycérol, de 0.01 à 2% en poids d'au moins un polymère poly(méth)acrylate, de 0.5 à 5% en poids d'au moins un polyéthylène glycol de masse moléculaire inférieure à 1000 g/mol, de 0.1 à 1.5% en poids d'octanediol, les pourcentages en poids étant exprimés par rapport au poids total de la composition.

2. Composition pharmaceutique selon la revendication 1, **caractérisée en ce qu'**elle comprend de 1 à 5% en poids d'au moins un polyéthylène glycol.

3. Composition pharmaceutique selon la revendication 1 ou 2, **caractérisée en ce que** le polyéthylène glycol a une masse moléculaire comprise entre 200 et 600 g/mol.

4. Composition pharmaceutique selon l'une des revendications précédentes, **caractérisée en ce qu'**elle comprend de 0.5 à 1.5% en poids de 1,2 octanediol.

5. Composition pharmaceutique selon l'une des revendications précédentes, **caractérisée en ce qu'**elle comprend, à titre de polymère poly(méth)acrylate, le mélange d'un polyméthacrylate de méthyle, de polymère réticulé acrylate/acrylate de C10-30 alkyle et de polyacrylate de sodium.

6. Composition pharmaceutique selon l'une des revendications précédentes, **caractérisée en ce que** le ratio massique entre la glycérine et l'octanediol est compris entre 5 :1 et 15 :1.

7. Composition pharmaceutique selon l'une des revendications précédentes, **caractérisée en ce qu'**elle contient de 5 à 10% en poids de glycérol.

8. Pansement comprenant une composition pharmaceutique selon l'une des revendications 1 à 7.

9. Composition pharmaceutique selon l'une des revendications 1 à 7 ou pansement selon la revendication 8, pour son utilisation dans le traitement des lésions cutanées aigües, telles que les plaies profondes, et le traitement des lésions cutanées chroniques, telles que les ulcères et les cicatrices.

10. Composition pharmaceutique selon l'une des revendications 1 à 7 ou pansement selon la revendication 8 pour son utilisation dans le traitement des ulcères dermiques chroniques, tels que les ulcères de pression, les ulcères des jambes et les ulcères du pied diabétique.

11. Composition pharmaceutique selon l'une des revendications 1 à 7 ou pansement selon la revendication 8 pour son utilisation dans le traitement de la cicatrisation des plaies occasionnées par une intervention chirurgicale.

12. Composition pharmaceutique selon l'une des revendications 1 à 7 ou pansement selon la revendication 8 pour son utilisation dans le traitement cicatrisant de lésions mucosales, telles que des fissures anales, des blessures en bouche, des crevasses occasionnées par l'allaitement, des crevasses situées sur les pieds ou les mains, et des boutons de fièvre.

13. Composition pharmaceutique selon l'une des revendications 1 à 7 ou pansement selon la revendication 8 pour son utilisation dans le traitement cicatrisant des piqûres de guêpes, d'abeilles, de taons, et de méduses.

14. Composition pharmaceutique selon l'une des revendications 1 à 7 ou pansement selon la revendication 8 pour son utilisation dans le traitement cicatrisant des dermites, des boutons de varicelle, des eczémas, des psoriasis, et des érythèmes fessiers.

15. Produit d'hygiène contenant moins de 0.1% en poids d'éthoxydiglycol et comprenant de l'eau, de 5 à 25 % en poids de glycérol, de 0.01 à 2% en poids d'au moins un polymère poly(méth)acrylate, de 0.5 à 5% en poids d'au moins un polyéthylène glycol de masse moléculaire inférieure à 1000 g/mol , de 0.1 à 1.5% en poids d'octanediol, les pourcentages en poids étant exprimés par rapport au poids total de la composition.

16. Produit d'hygiène selon la revendication 15 sous la forme d'un gel de rasage ou d'un baume après rasage pour peaux sensibles ou ultrasensibles, d'un décongestionnant nasal, ou d'un produit pour l'hygiène de l'oreille.

## Patentansprüche

1. Pharmazeutische Zusammensetzung zur topischen Anwendung auf der Haut oder den Schleimhäuten, enthaltend weniger als 0,1 Gew.-% Ethoxydiglycol und umfassend:
Wasser, 5 bis 25 Gew.-% Glycerin, 0,01 bis 2 Gew.-% wenigstens eines Poly(meth)acrylat-Polymers, 0,5 bis 5 Gew.-% wenigstens eines Polyethylenglykols mit einer molaren Masse von weniger als 1000 g/mol, 0,1 bis 1,5 Gew.-% Octandiol, wobei die Gewichtsprozentsätze bezogen auf das Gesamtgewicht der Zusammensetzung ausgedrückt sind.

2. Pharmazeutische Zusammensetzung nach Anspruch 1, **dadurch gekennzeichnet, dass** sie 1 bis 5 Gew.-% wenigstens eines Polyethylenglykols umfasst.

3. Pharmazeutische Zusammensetzung nach Anspruch 1 oder 2, **dadurch gekennzeichnet, dass** das Polyethylenglykol eine molare Masse zwischen 200 und 600 g/mol hat.

4. Pharmazeutische Zusammensetzung nach einem der vorhergehenden Ansprüche, **dadurch gekennzeichnet, dass** sie 0,5 bis 1,5 Gew.-% 1,2-Octandiol umfasst.

5. Pharmazeutische Zusammensetzung nach einem der vorhergehenden Ansprüche, **dadurch gekennzeichnet, dass** sie als Poly(meth)acrylat-Polymer die Mischung aus einem Polymethylmethacrylat, Acrylat/ C10-30-Alkylacrylat-Kreuzpolymer und Natriumpolyacrylat umfasst.

6. Pharmazeutische Zusammensetzung nach einem der vorhergehenden Ansprüche, **dadurch gekennzeichnet, dass** das Massenverhältnis zwischen Glycerin und Octandiol zwischen 5 : 1 und 15 : 1 liegt.

7. Pharmazeutische Zusammensetzung nach einem der vorhergehenden Ansprüche, **dadurch gekennzeichnet, dass** sie 5 bis 10 Gew.-% Glycerin umfasst.

8. Verband, umfassend eine pharmazeutische Zusammensetzung nach einem der Ansprüche 1 bis 7.

9. Pharmazeutische Zusammensetzung nach einem der Ansprüche 1 bis 7 oder Verband nach Anspruch 8, für ihre/seine Verwendung bei der Behandlung von akuten Hautschädigungen, wie tiefen Wunden, und der Behandlung von chronischen Hautschädigungen, wie Geschwüren und Narben.

10. Pharmazeutische Zusammensetzung nach einem der Ansprüche 1 bis 7 oder Verband nach Anspruch 8, für ihre/seine Verwendung bei der Behandlung von chronischen Hautgeschwüren, wie Druckgeschwüren, Geschwüren der Beine und Geschwüren des diabetischen Fußes.

11. Pharmazeutische Zusammensetzung nach einem der Ansprüche 1 bis 7 oder Verband nach Anspruch 8, für ihre/seine Verwendung bei der Behandlung der Wundheilung von durch einen chirurgischen Eingriff verursachten Wunden.

12. Pharmazeutische Zusammensetzung nach einem der Ansprüche 1 bis 7 oder Verband nach Anspruch 8, für ihre/seine Verwendung bei der wundheilenden Behandlung von Schleimhautschädigungen, wie Analfissuren, Verletzungen im Mund, durch das Stillen verursachten Rissen, auf den Füßen oder den Händen befindlichen Rissen und von Lippenherpes.

13. Pharmazeutische Zusammensetzung nach einem der Ansprüche 1 bis 7 oder Verband nach Anspruch 8, für ihre/seine Verwendung bei der wundheilenden Behandlung von Wespen-, Bienen-, Bremsen- und Quallenstichen.

14. Pharmazeutische Zusammensetzung nach einem der Ansprüche 1 bis 7 oder Verband nach Anspruch 8, für ihre/seine Verwendung bei der wundheilenden Behandlung von Hautentzündungen, Windpocken-Bläschen, Ekzemen, Schuppenflechten und Erythemen am Gesäß.

15. Hygieneprodukt, das weniger als 0,1 Gew.-% Ethoxydiglycol enthält und Wasser, 5 bis 25 Gew.-% Glycerin, 0,01 bis 2 Gew.-% wenigstens eines Poly(meth)acrylat-Polymers, 0,5 bis 5 Gew.-% wenigstens eines Polyethylenglykols mit einer molaren Masse von weniger als 1000 g/mol, 0,1 bis 1,5 Gew.-% Octandiol, wobei die Gewichtsprozentsätze bezogen auf das Gesamtgewicht der Zusammensetzung ausgedrückt sind, umfasst.

16. Hygieneprodukt nach Anspruch 15, in Form eines Rasiergels oder eines After Shave Balsams für sensible oder hochsensible Haut, eines Nasenschleimhautabschwellungsmittels oder eines Produkts für die Pflege des Ohrs.

## Claims

1. A pharmaceutical composition for topical application to skin or mucosa containing less than 0.1% by weight of ethoxydiglycol and comprising:
water, from 5 to 25% by weight of glycerine, from 0.01 to 2% by weight of at least one poly(meth)acrylate polymer, from 0.5 to 5% by weight of at least one polyethylene glycol having a molecular mass of less than 1000 g/mol , from 0.1 to 1.5% by weight of octanediol, the percentages by weight being expressed relative to the total weight of the composition.

2. The pharmaceutical composition as claimed in claim 1, **characterized in that** it comprises from 1 to 5% by weight of at least one polyethylene glycol.

3. The pharmaceutical composition as claimed in claim 1 or 2, **characterized in that** the polyethylene glycol has a molecular mass of between 200 and 600 g/mol.

4. The pharmaceutical composition as claimed in one of the preceding claims, **characterized in that** it comprises from 0.5 to 1.5% by weight of 1,2-octanediol.

5. The pharmaceutical composition as claimed in one of the preceding claims, **characterized in that** it comprises, as the poly(meth)acrylate polymer, a mixture of a methyl polymethacrylate, of a crosslinked acrylate/C10-30 alkyl acrylate polymer, and of a sodium polyacrylate.

6. The pharmaceutical composition as claimed in one of the preceding claims, **characterized in that** the mass ratio between glycerine and octanediol is between 5:1 and 15:1.

7. The pharmaceutical composition as claimed in one of the preceding claims, **characterized in that** it contains from 5 to 10% by weight of glycerine.

8. A dressing comprising a pharmaceutical composition as claimed in one of claims 1 to 7.

9. The pharmaceutical composition as claimed in one of claims 1 to 7 or the dressing as claimed in claim 8, for its use in the treatment of acute skin lesions, such as deep wounds, and the treatment of chronic skin lesions, such as ulcers and cicatrices.

10. The pharmaceutical composition as claimed in one of claims 1 to 7 or the dressing as claimed in claim 8, for its use in the treatment of chronic dermal ulcers, such as pressure ulcers, leg ulcers and diabetic foot ulcers.

11. The pharmaceutical composition as claimed in one of claims 1 to 7 or the dressing as claimed in claim 8, for its use in the treatment of healing of wounds caused by a surgical operation.

12. The pharmaceutical composition as claimed in one of claims 1 to 7 or the dressing as claimed in claim 8, for its use in the healing treatment of mucosal lesions, such as anal fissures, mouth injuries, cracks caused by breastfeeding, cracks located on the feet or the hands, and herpes labialis.

13. The pharmaceutical composition as claimed in one of claims 1 to 7 or the dressing as claimed in claim 8, for its use in the healing treatment of stings of wasp, bee, horsefly and jellyfish.

14. The pharmaceutical composition as claimed in one of claims 1 to 7 or the dressing as claimed in claim 8, for its use in the healing treatment of dermatitis, chicken pox spots, eczema, psoriasis and diaper rash.

15. A hygiene product containing less than 0.1% by weight of ethoxydiglycol and comprising water, from 5 to 25% by weight of glycerol, from 0.01 to 2% by weight of at least one poly(meth)acrylate polymer, from 0.5 to 5% by weight of at least one polyethylene glycol having a molecular mass of less than 1000 g/mol, from 0.1 to 1.5% by weight of octanediol, the percentages by weight being expressed relative to the total weight of the composition.

16. The hygiene product as claimed in claim 15, in the form of a shaving gel or an aftershave balm for sensitive or hypersensitive skin, a nasal decongestant or a product for ear hygiene.
